# EUROPEAN PATENT APPLICATION

(11) **EP 1 351 057 A1**
(43) Date of publication of application: **08.10.2003**
(21) Application number: 02007569.3
(22) Date of filing: 03.04.2002
(51) Int. Cl.: G01N 33/68

(54) **Inhibitors of caspase-3-mediated cleavage of essential ventricular myosin light chain**

(71) Applicant: Procorde GmbH, 82152 Martinsried (DE)
(72) Inventor: Moretti, Alessandra, 80805 München (DE); Holthoff, Hans-Peter, 82402 Seeshaupt (DE); Ungerer, Martin, 80799 München (DE); Münch, Götz, 80799 München (DE); Laugwitz, Karl-Ludwig, 80805 München (DE)
(74) Representative: Dendorfer, Claus, Dr.

(57) **Abstract**

The invention discloses use of a peptide containing an essential ventricular myosin light chain type 1 (vMLC1) amino acid sequence, which is functional as cleavage site for caspase-3, in the screening for a compound for the treatment of chronic or acute cardiovascular disease. Further, screening methods for inhibitors of the caspase-3-mediated cleavage of vMLC1 are disclosed.

## Description

### Field of the invention

The present invention relates to a novel use of a peptide containing an essential ventricular myosin light chain type 1 (vMLC1) amino acid sequence, which is functional as cleavage site for caspase-3. The peptide is preferably vMLC1. Moreover, the present invention relates to a cell-free *in vitro* screening method, a cell assay, and an *in vivo* assay for screening for inhibitors of the caspase-3-mediated cleavage of vMLC1. Preferably, the inhibitors are selective for inhibition of the caspase-3-mediated cleavage of vMLC1 over the caspase-3-mediated cleavage of a peptide at a conventional caspase-3 DEVD cleavage site. The present invention also relates to a kit-of-parts for identifying the inhibitors as well as novel inhibitors and medicines obtainable by the methods according to the invention.

### Background of the invention

Heart failure is a leading cause of mortality that ensues following the chronic activation of biomechanical stress pathways, resulting from various forms of myocardial injury (Chien, 2000). Histological evidence of apoptosis has been identified in several cardiovascular disorders leading to congestive heart failure (CHF) (Haunstetter and Izumo, 1998; Olivetti et al., 1997). Myocardial apoptosis represents a highly complex cell death program, whose execution is regulated by the caspase family of cystein proteases. Caspase-3 is a key effector enzyme and cleaves downstream critical cellular targets involved in chromatin condensation, DNA fragmentation, and cytoskeletal destruction, thereby expressing the dramatic morphological changes of apoptosis (Hengartner, 2000). Caspase-3 activation has been documented in the myocardium of end-stage heart failure patients (Narula et al., 1999), and caspase-3 expression is increased in patients with right ventricular dysplasia, a disease associated with progressive cell loss, and sudden death (Mallat et al., 1996). Recently, myocyte apoptosis, assessed by different biochemical hallmarks, including caspase-3 activity, has been described in pacing-induced heart failure models in animals, and correlates with the time-dependent deterioration of cardiac function (Cesselli et al., 2001; Laugwitz et al., 2001). Moreover, we showed that caspase-3 activation influences contractile performance of failing ventricular myocytes, and can be corrected *via* adenovirus-mediated gene delivery of the potent caspase inhibitor p35 with a positive impact on contractility (Laugwitz et al., 2001; Patent appl WO 01/60400). Moreover, single cardiomyocytes isolated from in vivo Ad-p35-infected failing hearts showed a significantly better average contractility (Laugwitz et al., 2001; Patent application WO 01/60400). On the other hand, injection of activated caspase-3 into cardiomyocytes causes an acute decrease of contractility (our patent appl WO 01/60400). These findings strongly suggest that non-nuclear, cytosolic effects are the basis for the beneficial effects of caspase-3 inhibition on cardiac contractility in heart failure. Therefore, extranuclear, cytosolic effects of caspase-3 were investigated in detail. The molecular mechanism by which activated caspase-3 causes a deterioration of cardiac function had not yet been established. In an attempt to answer this question, we performed a screening for caspase-3-interacting proteins expressed in the heart, using a modified yeast two-hybrid system. We identified vMLC1 as a novel target for active caspase-3, and investigated whether a cause-effect relationship between caspase-3 activation, vMLC1 cleavage and contractile performance exists in failing myocytes. As this was the case, a specific assay to screen for selective MLC1-protectants was established.

### Summary of the invention

The present invention is based on the recognition that the activation of caspase proteases involved in programmed cell death can mediate the cleavage of vital cytoskeletal proteins. Caspase-3 interacting proteins of the cardiac cytoskeleton were identified by a screening using a modified yeast two-hybrid system. Ventricular essential myosin light chain (vMLC1) was identified as a novel substrate for activated caspase-3. The cleavage of vMLC1 was demonstrated in failing myocardium *in vivo.* In myocytes isolated from failing ventricle, caspase-3 activation correlates with a morphological disruption of the organized vMLC1 staining of sarcomeres, and with a reduction in contractile performance. The present invention shows that direct cleavage of vMLC1 by activated caspase-3 contributes to depression of myocyte function by altering cross-bridge interactions between myosin and actin molecules. Therefore, activation of apoptotic pathways in the heart leads to contractile dysfunction prior to cell death.

Moreover, sequencing of cleaved vMLC1 led to the identification of a novel atypical cleavage site for caspase-3 (motif DFVE) which is not present in other caspase substrates. This atypical novel cleavage site was then used to establish a high throughput-scaleable *in vitro* assay to differentially screen for caspase-3 inhibitors which do not inhibit the physiological execution of cell death, but can protect vMLC1 from destruction in heart failure. Compounds identified in such a screen could be used to treat heart failure and other cardiac diseases, without having a prooncogenic potential.

Accordingly, the present invention provides a use of a peptide containing an essential ventricular myosin light chain type 1 (vMLC1) amino acid sequence which is functional as cleavage site for caspase-3 in the screening for a compound for the treatment of chronic or acute cardiovascular disease. The peptide may be any peptide that comprises the amino acid sequence DFVE. The peptide may e.g. be vMLC1. More preferably, the peptide may be DFVE advantageously having a group for chromogenic or fluorogenic detection of cleavage. Said amino acid sequence which is functional as cleavage site for caspase-3 is preferably DFVE.

In a preferred embodiment, the screening is directed to a compound which selectively inhibits the caspase-3-mediated cleavage of vMLC1 under predetermined conditions while essentially not inhibiting the caspase-3-mediated cleavage of a protein containing a functional caspase-3 DEVD cleavage site under the same conditions. The selectivity may be based on the structure of the compound. Further, the selectivity of the compound may be based on the concentration of the compound.

The present invention also provides a screening method for inhibitors of the caspase-3-mediated cleavage of vMLC1, which comprises:
(a) contacting a test compound and a sample containing
   (i) a peptide containing a vMLC1 amino acid sequence which is functional as cleavage site for caspase-3, and
   (ii) caspase-3,
      under predetermined conditions allowing cleavage of the peptide at the cleavage site in the absence of the test compound, followed by
(b) determining the presence or absence of an inhibition of the protein cleavage activity at the cleavage site as compared to the absence of the test compound, and
(c) identifying a compound as an inhibitor which provides for the presence of inhibition of the caspase-3-mediated cleavage of the protein in step (b).

Furthermore, the present invention provides a screening method for selective inhibitors of the caspase-3-mediated cleavage of vMLC1 over the caspase-3-mediated cleavage of a peptide containing a functional caspase-3 DEVD cleavage site, which comprises:
(a) contacting a predetermined amount of an inhibitor identified or identifiable by the above screening method and a sample containing
   (i) a peptide containing a functional caspase-3 DEVD cleavage site,
   (ii) caspase-3, and optionally
   (iii) a peptide containing a functional caspase-3 vMLC1 cleavage site, under predetermined conditions allowing cleavage of a peptide containing a functional caspase-3 vMLC1 cleavage site in the absence of the test compound, followed by
(b) determining the presence or absence of a change of the protein cleavage activity at the cleavage site of the peptide containing a functional caspase-3 DEVD cleavage site as compared to the absence of the test compound, and
(c) identifying a compound as a selective inhibitor which provides at the predetermined concentration for an essential absence of a change of the protein cleavage activity at the cleavage site of the peptide containing a functional caspase-3 DEVD cleavage site.

Preferably, the screening method is selective for inhibitors of the caspase-3-mediated cleavage of vMLC1 over the caspase-3-mediated cleavage of a peptide containing a functional caspase 3 DEVD cleavage site. The peptide containing a functional caspase-3 DEVD cleavage site used in the screening method for selective inhibitors is preferably of comparable size as the peptide containing a vMLC1 amino acid sequence used in the above screening method. The above two screening methods may be carried out simultaneously. They may be carried out in the same reaction vessel provided that cleavage at the DEVD cleavage site and at the DFVE cleavage site can both be monitored, e.g. by using fluorogenic peptides the cleavage of which gives rise to fluorescence signals which can be distinguished by wavelength. This embodiment enables a particularly efficient screening for inhibitors, since it allows to determine easily whether a test compound inhibits preferentially DEVD cleavage, preferentially vMLC1 cleavage, both cleavage types or whether it does not inhibit any cleavage type.

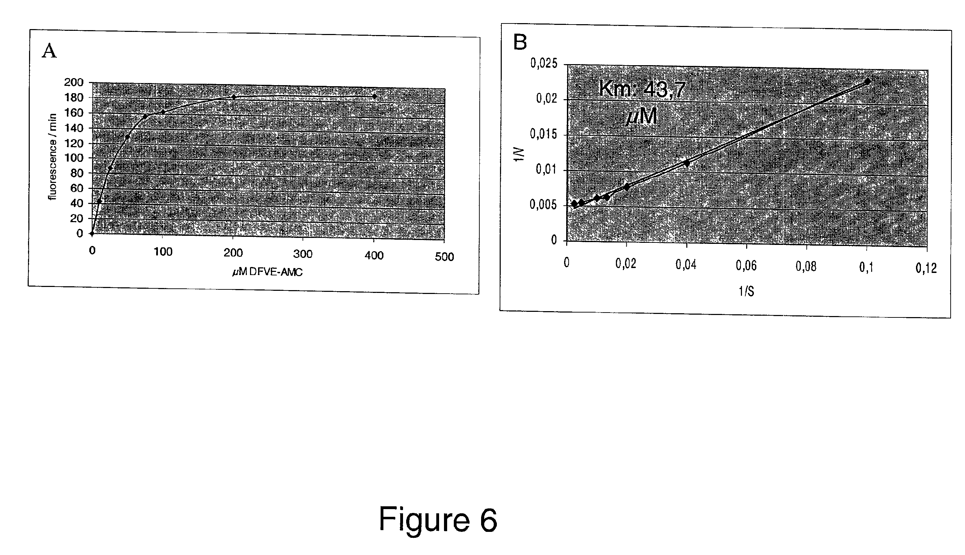

Detection of the cleavage activity in the above screening methods may be carried out by any known method. Detection of protein cleavage activity may comprise measuring the consumption of the peptide containing a vMLC1 amino acid sequence or of the peptide containing a functional caspase-3 DEVD cleavage site. Alternatively, the formation of a cleavage product may be measured. Measuring a cleavage product is preferred. These measurements are done after one or more time intervals after initiating the reaction. Small samples of the reaction mixture may be removed after said time interval(s) for analysis. In a more convenient screening method, the cleavage reaction may be followed continuously. The cleavage reaction may be followed by different means. Consumption of said peptide and/or formation of a cleavage product may e.g. be followed by chromatography, preferably by HPLC, and detection by retention volume and uv absorption. Further, mass spectroscopy, preferably ESI or MALDI spectroscopy, may be employed for this purpose. Most preferably, said peptide containing a vMLC1 amino acid sequence is designed such that its cleavage may be followed by a change of light absorption or a change of fluorescence (cf. example 8, Fig. 6) or FRET (fluorescence resonance energy transfer). The latter detection methods, notably those based on fluorescence detection, are also most suited for screening of a large number of test compounds like for high-throughput screening. Screening of a large number of test compounds is preferably done on multi-well plates. A test compound is tested at at least one concentration. Preferably the test compound is tested at various concentration in said screening method.

The present invention also provides a cell assay for screening for inhibitors of the caspase-3-mediated cleavage of vMLC1, which comprises
(a) providing a culture of isolated cardiomyocytes,
(b) introducing activated caspase-3 into cardiomyocytes of step (a),
(c) determining the presence or absence of a reduction of the extent of caspase-3-mediated cleavage of vMLC1 and/or an improvement of cell contractility under predetermined conditions in the presence of a test compound as compared to the absence of the test compound,
(d) identifying a compound as an inhibitor which provides for the presence of inhibition of the caspase-3-mediated cleavage of vMLC1 and/or which provides for an improved cell contractility in step (c).

It is preferred to determine the extent of caspase-3-mediated cleavage of vMLC1 in step (c).

The present invention also provides a cell assay for screening for selective inhibitors of the caspase-3-mediated cleavage of vMLC1 over the caspase-3-mediated cleavage of a peptide containing a functional caspase-3 DEVD cleavage site, which comprises
(a) providing a culture of isolated cardiomyocytes,
(b) introducing activated caspase-3 into cardiomyocytes of step (a),
(c) determining the presence or absence of a change of the extent of protein cleavage at the cleavage site of the peptide containing a functional caspase-3 DEVD cleavage site in the presence of a predetermined amount of an inhibitor identified or identifiable by one of the assays described above, preferably by the above cell assay, as compared to the absence of the inhibitor, and
(c) identifying a compound as a selective inhibitor which provides in the predetermined amount for an essential absence of a change of the protein cleavage activity at the cleavage site of the peptide containing a functional caspase-3 DEVD cleavage site.

A culture of isolated cardiomyocytes may be provided according to known methods (cf. example 5; Laugwitz et al., 1999). Said cardiomyocytes are preferably of mammalian origin, e.g. from mice, rats, rabbits, pigs etc. More preferably, said cardiomyocytes are of human origin. Activated caspase-3 is preferably introduced into said cardiomyocytes by direct injection. Further, caspase-3- may be introduced into cardiomyocytes on a vector containing a caspase-3 gene under the control of a promoter. Said cardiomyocytes may be adenovirally transfected. Alternatively, a culture of isolated cardiomyocytes which express activated caspase-3, preferably constitutively, may be used. For certain test compounds, providing said cardiomyocytes with said test compound may require means to overcome a limited cell membrane permeability for said test compound. The membrane permeability may be increased by various known means. The above two cell assays may be carried out simultaneously. Cells other than cardiomyocytes may be used in the above cell assays provided they contain vMLC1.

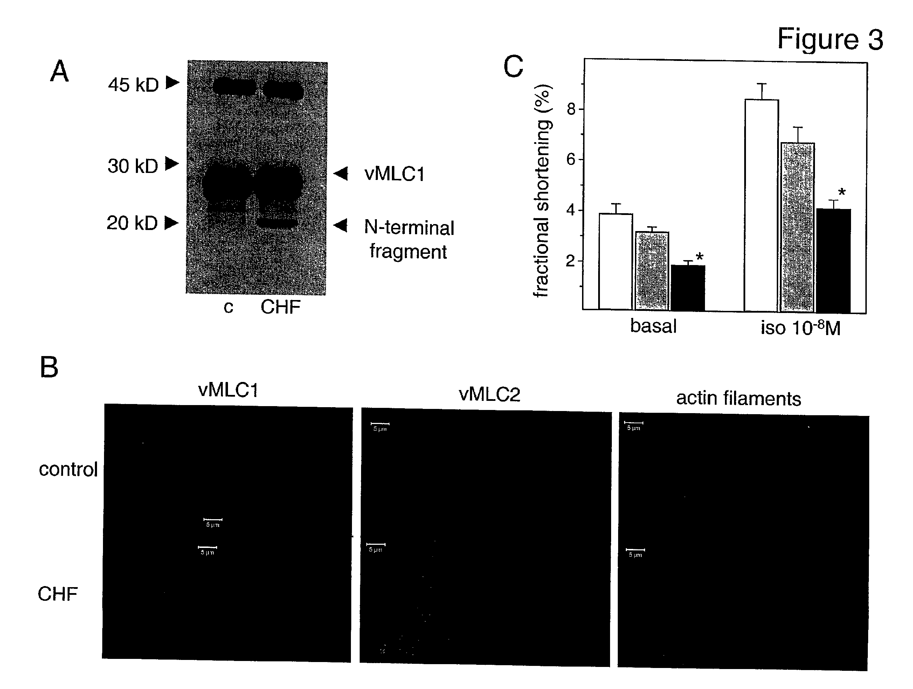

In said cell assay for screening for inhibitors, detection of the extent of protein cleavage may be done by Western blotting e.g. as described in example 3. Also, immunoprecipitation may be employed (see example 4). Preferably, the presence or absence of the specific ≈20 kDA cleavage product of vMLC1 is determined in said cells (see Fig. 3A). A further possibilty to determine the action of a test compound on said cell is to measure an improvement of cell contractility. Preferably, a test compound is identified as inhibitor by finding less of said specific ≈20 kDA cleavage product of vMLC1 in the presence of said test compound than in its absence.

The invention further provides an *in vivo* assay for screening for inhibitors of the caspase-3-mediated cleavage of vMLC1, which comprises
(a) providing an animal model, preferably for heart failure,
(b) administering a test compound to the animal model of step (a),
(c) determining the presence or absence of a reduction of the extent of caspase-3-mediated cleavage of vMLC1 and/or determining an improvement of heart failure under predetermined conditions in the presence of the test compound as compared to the absence of the test compound,
(d) identifying a compound as an inhibitor which provides for the presence of inhibition of the caspase-3-mediated cleavage of vMLC1 and/or which provides for an improvement of heart failure in step (c).

It is preferred to determine the presence or absence of a reduction in the extent of caspase-3-mediated cleavage of vMLC1 in step (c).

An *in vivo* assay is further provided for screening for selective inhibitors of the caspase-3-mediated cleavage of vMLC1 over the caspase-3-mediated cleavage of a peptide containing a functional caspase-3 DEVD cleavage site, which comprises
(a) providing an animal model, preferably for heart failure,
(b) administering a test compound to the animal mode! of step (a),
(c) determining the presence or absence of a change of the extent of protein cleavage at the cleavage site of the peptide containing a functional caspase-3 DEVD cleavage site in the presence of a predetermined amount of an inhibitor identified or identifiable by an assay described above, preferably by said in vivo assay described above, as compared to the absence of the inhibitor, and
(d) identifying a compound as a selective inhibitor which provides in the predetermined amount for an essential absence of a change of the protein cleavage activity at the cleavage site of the peptide containing a functional caspase-3 DEVD cleavage site.

In said *in vivo* assay for screening for inhibitors, the determination in step (c) may be based on measuring the contractility of cardiomyocytes (cf. example 9). Preferably, step (c) is based on determining, e.g. clinically, an improvement of heart failure. Most preferably, the presence or absence of a reduction in the extent of caspase-3-mediated cleavage of vMLC1 is determined. This may be done by Western blotting and determination of said specific ≈20 kDA cleavage product of vMLC1 as described above for said cell assay.
An animal model of heart failure may be created by rapid pacing (cf. example 5).

Said screening method, said cell assay and said in vivo assay of the invention differ in complexity and in the reliability of the obtained result for an in vivo situation. Said screening method is best suited for testing a large number of test compounds. Said in vivo assay may provide data which are close to an in vivo situation. Said cell assay and said in vivo assay may further provide toxicological data of a test compound. It is therefore preferred to carry out said screening method first, preferably with many test compounds. An inhibitor identified in said screening method may then be used in said cell assay. A test compound used in said in vivo assay has preferably been identified as an inhibitor in said screening method and most preferably in said cell assay. A test compound found to inhibit caspase 3-mediated cleavage of a peptide containing a functional caspase 3 vMLC1 cleavage site is preferably tested for its cleavage acitivity of a peptide containing a functional caspase 3 DEVD cleavage site on each stage (the stage of the screening method, the stage of the cell assay and the stage of the in vivo assay). Most preferably, a test compound not or weakly inhibiting caspase-3-mediated cleavage of a DEVD cleavage site in said screening method for selective inhibitors or in said cell assay for screening selective inhibitors is used in said cell assay or said in vivo assay, respectively.

The present invention also provides a kit-of-parts for identifying inhibitors of the caspase-3-mediated cleavage of vMLC1, comprising the following components:
(i) a first component comprising a peptide containing an essential ventricular myosin light chain amino acid sequence, which is functional as cleavage site for caspase-3, and
(ii) a second component comprising caspase-3.

The present invention also provides a kit-of-parts for identifying selective inhibitors of the caspase-3-mediated cleavage of vMLC1 over the caspase-3-mediated cleavage of a peptide containing a functional caspase-3 DEVD cleavage site, comprising the following components:
(i) a first component comprising a peptide containing a functional caspase-3 DEVD cleavage site,
(ii) a second component containing caspase-3, and optionally
(iii) a third component comprising a peptide containing a functional caspase-3 vMLC1 cleavage site.

The present invention also provides an inhibitor of caspase-3-mediated cleavage of essential ventricular myosin light chain obtained or obtainable by the method of the invention. Preferably, the inhibitor is a selective inhibitor of the caspase-3-mediated cleavage of vMLC1 over the caspase-3-mediated cleavage of a peptide containing a functional caspase-3 DEVD cleavage site. The present invention also provides a use of the inhibitor for the preparation of a medicament for the treatment of chronic or acute cardiovascular disease.

Said inhibitor may be a peptide inhibitor or a non-peptide inhibitor. An inhibitor may be identified by screening large libraries of chemical compounds which may be prepared by combinatorial chemistry (see e.g. Anal. Chem. 69 (1997) 2159-2164, or Anticancer Drug Res. 12 (1997) 145-167) or which are commercially available. Further, an inhibitor of caspase-3-mediated cleavage of a vMLC1 amino acid sequence may be designed based on the DFVE amino acid sequence. Modifications of the DFVE moiety may be performed which have a high likelihood of not preventing binding to caspase-3 but which have a high likelihood of not being cleavable by caspase-3. The peptide bond of the DFVE moiety cleaved by caspase-3 may preferably be modified by substituting atoms of said peptide bond by other atom types. For example, the peptide nitrogen atom or the carbonyl oxygen atom may be replaced by a carbon atom. Such a modified compound may then be used as a test compound in the assays of the invention.

Further known caspase-3 inhibitors may used in the assays of the invention and as starting inhibitors to be improved. Preferably, known caspase-3 inhibitors are used which have a moderate inhibition capability for DEVD cleavage as opposed to strong caspase-3 inhibitors. Such moderate inhibitors may inhibit DFVD cleavage but not DEVD cleavage by caspase-3 at a certain concentration due to the differing affinities of caspase-3 for these cleavage sites (see below). Caspase-3 inhibitors are described e.g. in WO019435 WO019007 WO0220465, and in US6,355,618.

Further, rational drug design may be conducted based on the protein structure of caspase-3 and the cleavage site of a vMLC1 amino acid sequence. Also, the properties of an inhibitor identified in an assay of the invention may be altered or improved by rational drug design. The crystal structure at 2.7 Å resolution of the complex of caspase-3 and the inhibitor XIAP has been published (Riedl *et al*., Cell, 104 (2001) 791-800) and the coordinates of the structure are available from the Protein Data Base (entry 1130). Based on the 3-D structure of caspase-3, a potential inhibitor can be examined through the use of manual computer modeling or by using a standard docking program such as GRAM, DOCK, or AUTODOCK (Goodsell et al. (1990) Proteins: Structure, Function and Genetics, 8, 195-201; Kuntz et al. (1982) J. Mol. Biol. 161, 269-288). Programs usable for computer modelling include Quanta (Molecular Simulations, Inc.) and Sibyl (Tripos Associates). This procedure can include computer fitting of a potential inhibitor to the active site of caspase-3. Computer methods can also be employed to estimate the attraction, repulsion, and steric hindrance of a potential inhibitor to the active site of caspase-3. Generally, the tighter the fit (e.g., the lower the steric hindrance, and/or the greater the attractive force), the more potent the potential drug will be since these properties are consistent with a tighter binding constant. Furthermore, the higher the specificity of a potential inhibitor, the more likely it is that it will not interfere with related proteins, thereby minimizing potential side-effects due to unwanted interactions with other proteins.

### Short Description of the Figures

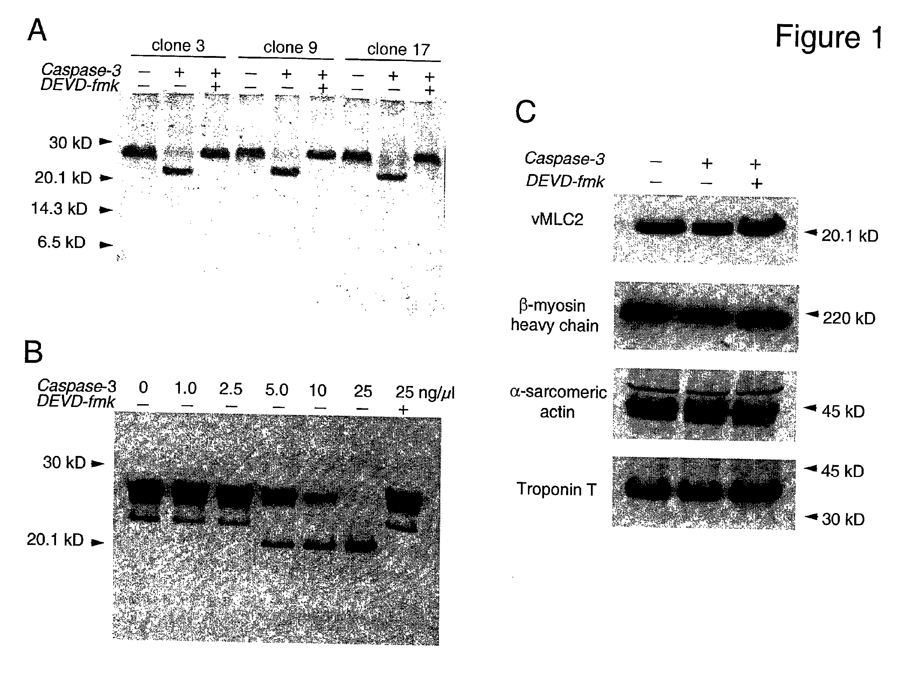

**Figure 1.** *In vitro* cleavage of vMLC1 by recombinant active caspase-3. **(A)** SDS-15% PAGE of biotinylated lysine-labeled proteins from 3 positive clones encoding human vMLC1 after 1 h incubation with 15 ng/µl human recombinant active caspase-3 in presence or absence of 25 µM DEVD-fmk. **(B)** Immunoblot analysis of native vMLC1 cleavage in protein extracts from rabbit left ventricle, incubated with indicated concentration of recombinant active caspase-3 for 1 h. DEVD-fmk was applied in a concentration of 25 µM. **(C)** Same protein extracts as in *B* after 1 h incubation with 25 ng/µl recombinant active caspase-3 and with or without 25 µM DEVD-fmk. Immunoblot analysis using monoclonal antibodies against vMLC2, cardiac α/β myosin heavy chain, α-sarcomeric actin and troponin T.

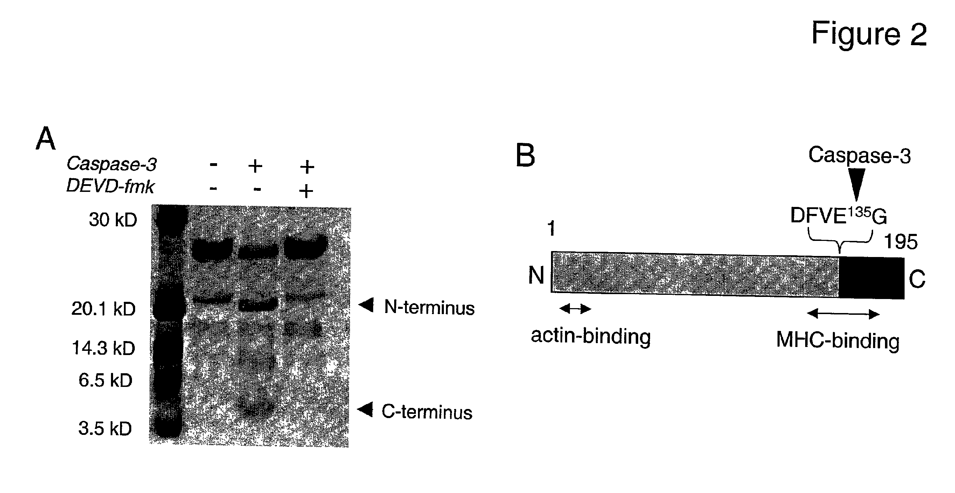

**Figure 2.** Determination of the cleavage site of human vMLC1. **(A)** Cleavage of purified human vMLC1 (10 µg) by human recombinant active caspase-3 (20 ng/µl, 1 h, 37°C), analyzed by SDS-16.5% PAGE and Coomassie blue staining. Bands corresponding to the N-terminal and C-terminal fragments are indicated. **(B)** A diagram showing the caspase-3 cleavage site at E¹³⁵ of human, rabbit, rat and chicken vMLC1.
**Figure 3.** *In vivo* cleavage of vMLC1 in failing myocardium and its effect on myocyte contractile performance. **(A)** Immunoblot analysis after immunoprecipitation of native vMLC1 cleavage products in extracts of left ventricle from control healthy (c) and failing (CHF) rabbit myocardium. Shown are representative data from one of three animals in each group. **(B)** Laser scanning fluorescence microscopy of representative ventricular rabbit cardiomyocytes isolated from the anterolateral wall of control and failing myocardium. Green fluorescence (520 nm) shows activated caspase-3 identified by FAM-DEVD-fmk, blue fluorescence (420 nm) illustrates nuclei by Hoechst 33258, and red fluorescence (615 nm) reflects vMLC1 (a-d panels), or vMLC2 (e-h panels), or polymeric actin by phalloidin staining (i-l panels). Scale bars, 5 µm. One hundred cells isolated from 3 animals were analysed in each group. **(C)** Contraction amplitude under basal conditions and isoproterenol stimulation (10⁻⁸M) measured in single left ventricle cardiomyocytes. White columns: cells from control myocardium; gray columns: cells from failing myocardium negative for activated caspase-3; black columns: cells from failing myocardium positive for activated caspase-3. Data are expressed as mean ± SEM, and analysed by one way analysis of variance (ANOVA) followed by Scheffe post-hoc analysis. *n* = 40 cells from 3 animals in each group. **p* < 0.005, ***p* < 0.001 in comparison with control cells (basal, isoproterenol 10⁻⁸M).

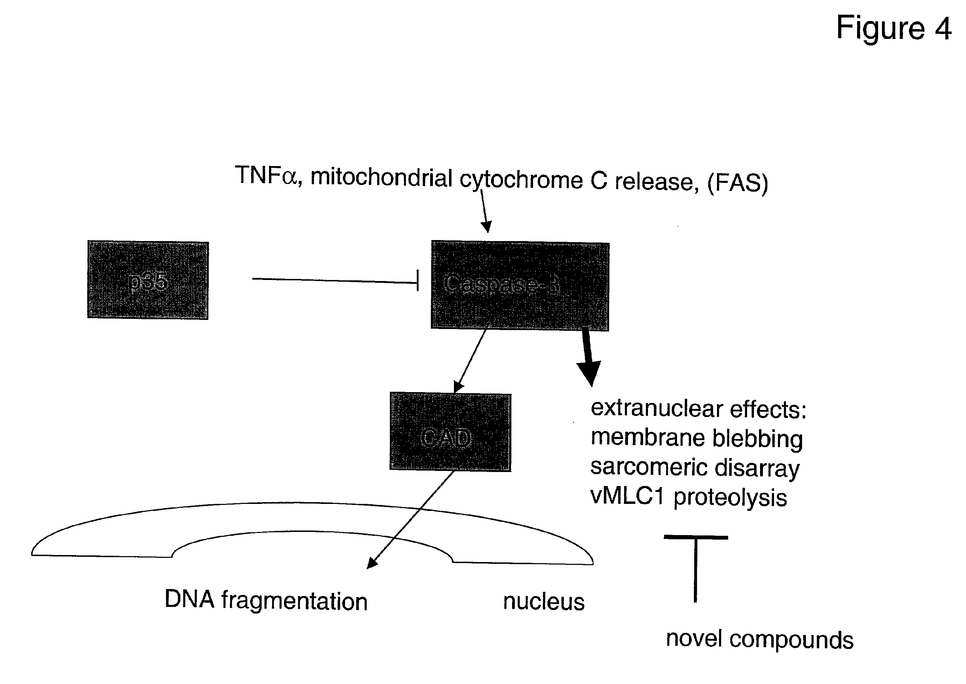

**Figure 4:** Summary of the nuclear and extranuclear effects of caspase-3 in the failing heart.

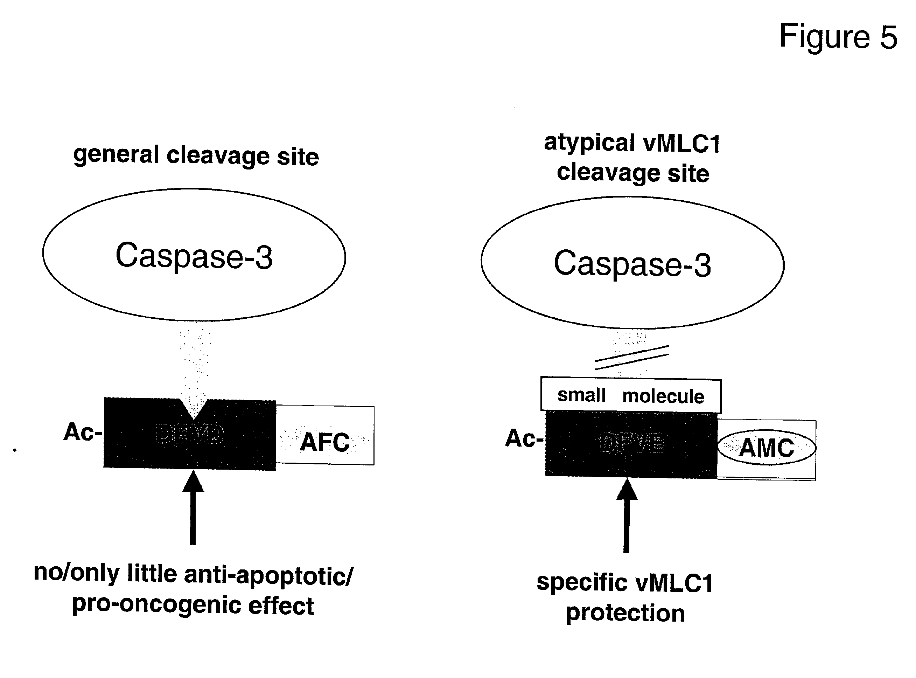

**Figure 5:** Assay for novel inhibitors by differential inhibition. Principle of a peptide-based screening assay to identify selective vMLC1 protectants which do not block the physiological execution of nuclear apoptosis. Such compounds inhibit the cleavage of the indicator peptide DFVE, but not of DEVD.
**Figure 6.** Determination of Km for DFVE-AMC. **(A)** Kinetic analysis and Lineweaver-Burk plot of DFVE cleavage by caspase-3. Ac-DFVE-AMC is a synthetic tetrapeptide substrate that is cleaved by recombinant human caspase3 (3nM). This substrate is cleaved between E and AMC (7-amino-4-methylcoumarin), releasing the fluorogenic AMC, which is detected by spectrofluorometry. **(B)** Lineweaver-Burk plot of the reaction

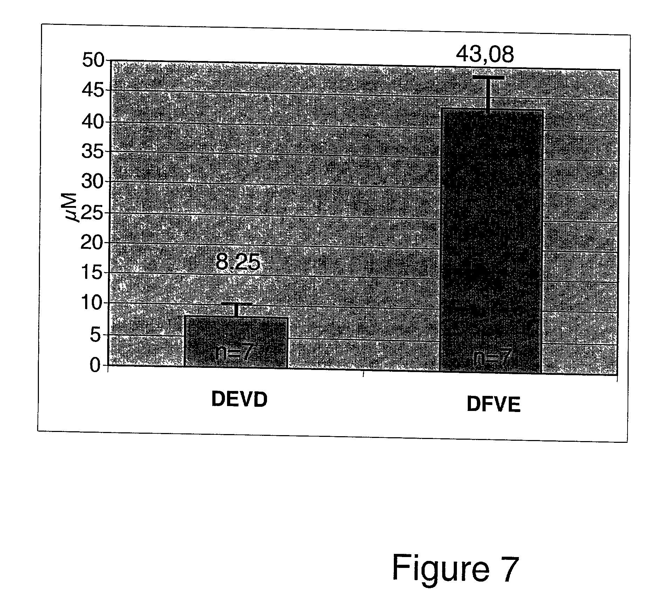

**Figure 7.** Specificities for caspase-3. Comparison of K_{M} values for DFVE and DEVD cleavage by caspase-3. K_{M}-values of Ac-DFVE-AMC and Ac-DEVD-AMC were determined.

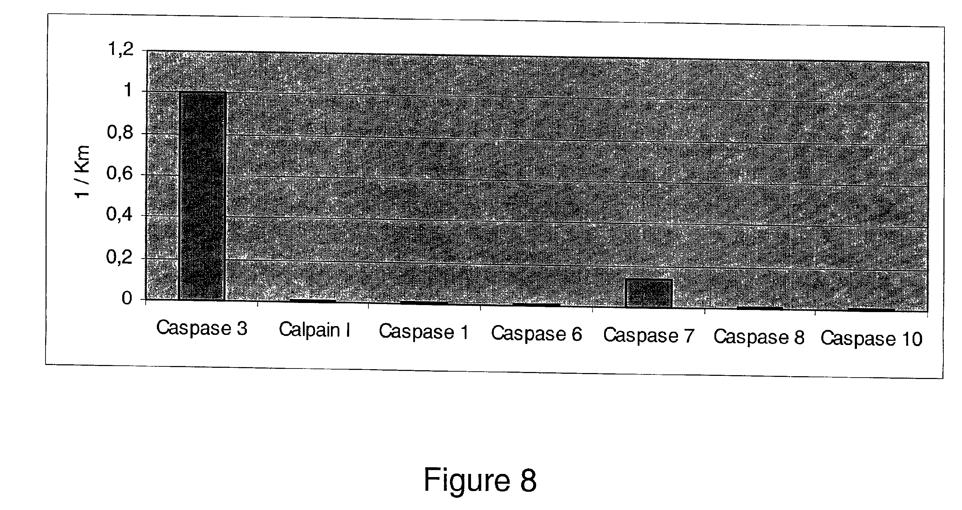

**Figure 8:** Effect of different proteases - other caspases and calpain - on cleavage of the peptide substrate Ac-DFVE-AMC. The 1/K_{M}-value for caspase-3 was normalized to 1, and 1/K_{M}-values of other caspases/proteases are shown in relation to that value. Whereas caspase-3 and 7 showed clear DFVE substrate cleavage, although with different affinity, no such cleavage was observed with the other caspases. The functional proteolytic activity of all proteases used was confirmed with the typical peptide substrate of each protease.

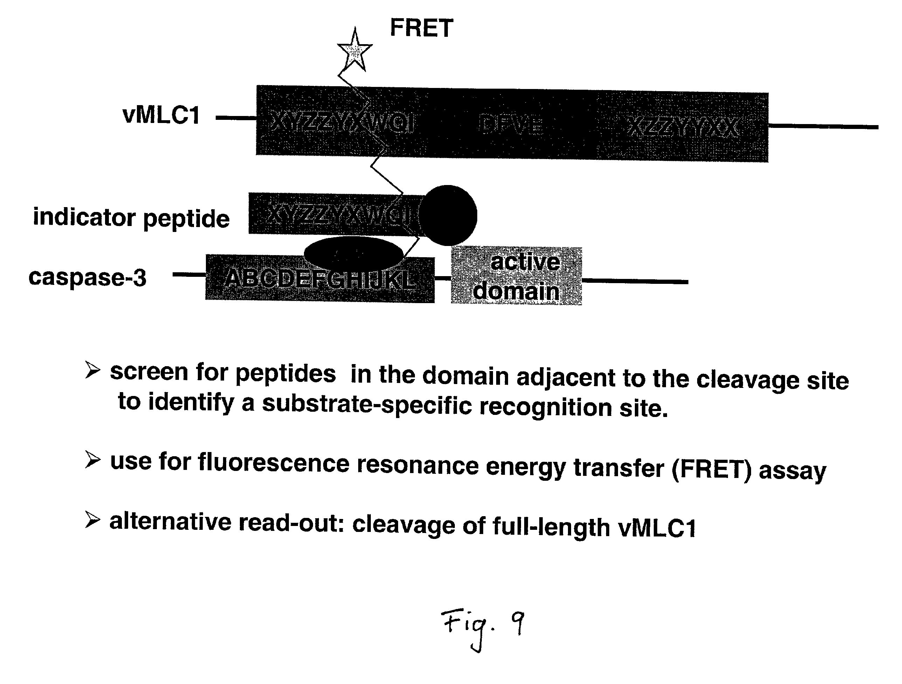

**Figure 9:** Concept for an alternative assay to screen for substrate-specific inhibitors of caspase-3. By peptide mapping, functionally relevant regions in the domain adjacent to the active binding pocket are identified, and used for an alpha screen with FRET technology.

### Detailed Description of the Invention

### Identification of vMLC1 as substrate for caspase-3

In a rabbit model of CHF obtained by rapid ventricular pacing, we previously demonstrated that caspase-3 activation is associated with a reduction in contractile force of failing myocytes. Using *in vivo* transcoronary adenovirus-mediated gene delivery of the potent caspase inhibitor p35, we could correct caspase-3 activation in failing myocardium with a positive impact on sarcomeric organization and contractile performance (Laugwitz et al., 2001). The beneficial effect was observed at the level of the intact heart in vivo, but also at the level of single cells isolated from in vivo Ad-p35-infected myocardium. Therefore, extranuclear, cytosolic mechanisms independent of the execution of nuclear apoptosis must have mainly accounted for the negative effects of caspase-3 activation in heart failure. To better understand the mechanism that may cause cytosolic caspase-3-mediated sarcomeric disarray, we performed a screening for caspase-3 interacting proteins expressed in the heart. We employed a modified yeast two-hybrid system utilizing, as bait vector, the plasmid pBTM-casp3-p12p17^{m}, which has already been succesfully used to identify gelsolin as a substrate for caspase-3 (Kamada et al., 1998). Both large (p17) and small (p12) subunits of active caspase-3 were separately expressed in yeast at equimolar ratios under ADH1 promoters. The small subunit was fused to the LexA DNA-binding domain, and a point mutation in the active site of the enzyme (Cys-163 to Ser) prevented proteolytic cleavage of interacting substrates. The bait plasmid was cotransfected into yeast with a human heart cDNA expression library fused to the Gal4 activation domain. By screening 30 millions transformants, we obtained 125 positive clones which were divided into 22 groups on the base of inserted fragment size and restriction enzyme digestion pattern. DNA sequencing analysis showed that six of the positive clones encoded overlapping C-terminal parts (clone #7, #12, and #20) or the complete sequence (clones #3, #9, and #17) of vMLC1. MLC1 is one of the six polypeptide chains of the myosin molecule, and is proposed to function as an actin/myosin tether regulating cross-bridge cycling events (Morano, 1999). In this study we further analysed the vMLC1 clones, and the others will be described elsewhere.

To examine cleavage of vMLC1 candidates by caspase-3 *in vitro,* proteins encoded by the cDNAs were produced by *in vitro* transcription/translation-reaction. As shown in Figure 1 A, clones #3, #9 and #17, which contained the complete sequence of human vMLC1, were cleaved by human recombinant active caspase-3, and this cleavage was blocked in the presence of its tetrapeptide inhibitor DEVD-fmk, suggesting that vMLC1 is a substrate for caspase-3. Immunoblot analysis of protein extracts from left ventricle, incubated with active caspase-3, confirmed this result (Fig. 1 B). A ∼20 kD cleavage product for vMLC1 was already evident with 5 ng/µl active caspase-3. Indeed, other structurally related sarcomeric proteins, ventricular regulatory myosin light chain (vMLC2), or β myosin heavy chain, were not cleaved, demonstrating that cleavage of vMLC1 was not due to a generalized degradation of proteins (Fig. 1 C).

To determine caspase-3 cleavage site of vMLC1, purified human vMLC1 was incubated with recombinant active enzyme (Figure 2 A). Cleavage of purified vMLC1 resulted in two fragments at ∼20 kD and ∼5 kD. Edman sequence analysis of the cleavage products revealed that caspase-3 cleaved vMLC1 at E¹³⁵ of the C-terminal motif DFVE¹³⁵G, which is highly conserved (Fig. 2 B). This result was confirmed by immunoblot analysis, using a monoclonal antibody for vMLC1 (clone F109.16A12) directed against the sequence V¹³⁴EGLRV¹³⁹ at the caspase-3 cleavage site. The antibody detected the intact vMLC1 protein but did not detect either of the two cleavage fragments (data not shown). The mapped cleavage site corresponds to the caspase-3 consensus sequence DXXD (Cohen, 1997) with exception of substituting the last aspartate residue for the similar acidic glutamate residue at position 135. Recently, cleavage of lens connexin 45.6 by caspase-3 has also been identified at the E³⁶⁷ residue of DEVE³⁶⁷G (Yin et al., 2001). An extensive screening of several databases did not, however, show any other known substrate of caspase-3 to be cleaved at the same, atypical cleavage site (motif DFVE).

### Disruption of vMLC1 and sarcomere integrity in vivo

To determine the functional relevance of vMLC1 cleavage by caspase-3 in the heart *in vivo,* we investigated the evidence of vMLC1 cleavage products in extracts from rabbit failing ventricular myocardium, where we have previously documented a ∼6-fold increase in caspase-3 activity (Laugwitz et al., 2001). As shown in Figure 3 A, the intact vMLC1 protein of ∼27 kD was relatively stable in healthy control hearts. In contrast, a main ∼20 kD fragment, corresponding to the N-terminal cleavage product, was present in failing myocardium.

Myosin is the major component of the thick filaments of sarcomeres, and consists of two heavy chains (α and β), each associated with two types of light chains, the essential (MLC1) and the regulatory (MLC2). X-ray crystallographic analyses demonstrated that essential and regulatory myosin light chains are spatially close, and are both associated with the neck region of the myosin heavy chain globular head (Rayment et al., 1993). To examine whether in failing myocytes a morphological disruption of the organized vMLC1 staining of A-bands in sarcomeres occurred and whether it correlated with caspase-3 activation, single cardiomyocytes from control and CHF hearts were isolated. Fig. 3 B shows confocal laser scanning microscopy of isolated ventricular myocytes after staining for activated caspase-3 and immunostaining for vMLC1 or vMLC2. In cardiomyocytes isolated from control hearts, there was no evidence of caspase-3 activation, and both myosin light chains appeared organized in the sarcomeric units (Fig. 3 B, panels a-b and e-f). In contrast, failing myocytes with activated caspase-3 presented a loss of the characteristic localization of vMLC1 in sarcomeres, and the A-band vMLC2 staining, which was maintained, showed a reduced sarcomeric organization compared to that of control cells (Fig. 3 B, panels c-d and g-h). Sarcomeric disarray in failing cells presenting caspase-3 activation was confirmed by phalloidin staining, which visualizes actin filaments (Fig. 3 B, panels k-l). Single-cell shortening experiments in failing cardiomyocytes showed a reduction of basal and isoproterenol-stimulated contraction correlated to the amount of caspase-3 activation in the cytosol of the failing cells (Fig. 3 C).

A lot of data suggests that myosin light chains play an important role in cardiac and skeletal muscle function. Removing MLCs from chicken skeletal muscle myosin reduces the velocity of actin filament movement by 90% without significant loss of the myosin ATPase activity in an *in vitro* motility assay (Lowey et al., 1993). Furthermore, MLC2 removal has little effect on isometric force, whereas MLC1 removal reduces the isometric force by over 50% (VanBuren et al., 1994). Mutations in the human essential light chain (Met149Val) or regulatory light chain (Glu22Lys, Pro94Arg) of myosin are associated with rare variants of inherited cardiac hypertrophy, characterized by midventricular cavity obstruction, and correlate with disruption of the stretch activation response of the cardiac papillary muscles (Potter et al., 1996). Transgenic mice expressing the human mutant MLC1^{Met149Val} faithfully replicate the cardiac disease of the patients with this mutant allele (Vemuri et al., 1999).

In the human heart, two different essential myosin light chain isoforms exist: **(a)** an atrial specific isoform (aMLC1), which is expressed in the fetal heart and decreases to undetectable levels during early postnatal development in the ventricle, but persists in the atrium for the whole life, and **(b)** a ventricular specific isoform (vMLC1), which is the same isoform present in adult slow skeletal muscle (Price et al., 1980). The reexpression of aMLC1 in adult human ventricles has been reported in patients with ischemic or dilative cardiomyopathy and valvular heart disease (Schaub et al., 1987; Sütsch et al., 1992). Interestingly, in such patients with end-stage heart failure caspase-3 activation has also been documented (Narula et al. 1999). The isoform shift of vMLC1 to aMLC1 correlates with an increase in cross-bridge cycling kinetics as measured in skinned fibers derived from the diseased muscle (Morano et al., 1997). Postsurgical return to a normal hemodynamic state decreases aMLC1 expression in these patients (Sütsch et al., 1992). The functional significance of this isoform switch is not completely clear, but may be a direct compensatory mechanism to caspase-3 induced vMLC1 cleavage, triggered when the heart attempts to maintain normal cardiac function.

The molecular mechanism for MLC1 to affect the cross-bridge kinetics seems to reside in its Ala-Pro-rich extended N-terminus, which has been shown to interact with the C-terminus of actin (Trayer et al., 1987; Milliganetal., 1990). The extended MLC1 N-terminus may provide a tether between the myosin and actin filaments, serving to position the two filament systems for cross-bridge interaction and to amplify small movements of the myosin globular head (Sweeney 1995). The MLC1 C-terminus anchors the protein to the myosin globular head. Destruction of vMLC1 at the C-terminal motif DFVE¹³⁵G by activated caspase-3 could alter myosin/actin cross-bridge interactions by modifying myosin head stability and thereby lead to reduced force transmission. Taken together, this data clearly illustrates that minute changes in vMLC1 structure or composition, particularly in the C terminal anchoring moiety of vMLC1, can have a dramatic impact on myocyte function and heart contractility.

We have therefore demonstrated that vMLC1 is a cellular target for activated caspase-3. vMLC1 is cleaved, and its localization in sarcomeres is partially lost in failing cardiomyocytes, presenting caspase-3 activation and reduced contractile performance. It is plausible that vMLC1 disruption could alter the stiffness of the myosin neck region and therefore reduce the full range of myosin movement during contraction. Our findings suggest that caspase-3-mediated cleavage of vMLC1 may represent a molecular mechanism contributing to the deterioration of cardiac function prior to myocyte cell death (summarized in Figure 4).

As we had found that the atypical vMLC1 cleavage site DFVE did not occur in any other known substrate of caspase-3, we intended to identify specific MLC protectants for the treatment of heart failure. The atypical novel cleavage site was therefore used to establish a high throughput-scaleable in vitro assay to differentially screen for caspase-3 inhibitors which do not inhibit the physiological execution of cell death, but can protect vMLC1 from destruction in heart failure (summarized in Figure 5). Compounds identified in such a screen could be used to treat heart failure and other cardiac diseases, without having a pro-oncogenic potential.

To this end, fluorimetric in vitro assays using the specific cleavage sites coupled to indicator dyes were established. Caspase-3 and the structurally almost identical caspase-7 (Wei et al., 2000) cleaved both substrates, DEVD and DFVE, with a highly reproducible Michaelis-Menten kinetic. Fig. 6 shows an example of the kinetic course and a Lineweaver-Burk plot for the substrate DFVE cleaved by caspase-3. To our surprise, we found that the K_{M}-values for caspase-3 induced cleavage of both substrates differed markedly. We measured a K_{M}-value for DEVD which compared well to existing literature data (Table 1), whereas the K_{M}-value for DFVE was in the range of some other rare substrates of caspase-3 (Table 1). This differential substrate affinity allows to screen for specific inhibitors of vMLC1 cleavage, which would, however, not affect the cleavage of most other known substrates of caspase-3 and 7. In such a screen, every compound would be tested at identical concentrations for the inhibition of both reactions (first assay), and compounds which specifically inhibit DFVE, but not DEVD cleavage would be selected and tested in cardiomyocytes ex vivo upon coinjection of active caspase-3 for their capacity to protect against vMLC1 cleavage (2nd assay).

As p35 inhibits all effector caspases (subtypes 1,3,6,7,8 and 10; e.g., Zhou et al., Biochemistry 1998; 37:10757), our concern was whether the observed beneficial effects of p35 in heart failure were really due to inhibition of caspase-3 and of the structurally almost identical caspase-7. Especially, we sought to exclude that the decrease in vMLC1 cleavage in the presence of p35 was due to predominant inhibition of a different caspase, and to exclude that the cleavage of vMLC1 was induced by caspase-3 only indirectly acting via other effector caspases. Therefore, we tested all these enzymes for their capacity to cleave Ac-DFVE-AMC. We found that only caspase-3 and 7, which are structurally very similar, can specifically cleave this substrate, whereas no cleavage was detectable with other caspases nor with the related protease calpain-I at physiological substrate concentrations. However, the highly homologous caspase-7 cleaved the new substrate DFVE with almost 1 log scale lower affinity compared to caspase-3.

**Table I**

| Kinetic constants for chromogenic peptide substrates (J Biol Chem. 1997 Apr 11; 272(15):9677-82) | |
|---|---|
| Substrate | Km (µM) |
| Ac-DEVD-pNA | 11 |
| Ac-DQMD-pNA | 44 |
| Ac-VDVAD-pNA | 67 |
| Ac-VEID-pNA | 250 |
| Ac-YEVD-pNA | 370 |
| Ac-VQVD-pNA | 510 |

### Examples

### Example 1: Yeast Two-Hybrid Screening

Yeast two-hybrid screening using pBTM-casp3-p12p17^{m} as bait vector was performed with a human heart cDNA library, fused to the Gal4 activation domain in the pACT2 plasmid (Clontech, Heidelberg, Germany), following the Hybrid Hunter two-hybrid system protocol (Invitrogen, Groningen, The Netherlands) in L40 yeast cells (*MATa trp1 lue2 his3 ade2 LYS2::4lexAop-HIS3 URA3::8lexAop-lacZ*). A total of 30x10⁶ independent clones were screened by selective growth on Trp⁻ /Leu⁻ /His⁻ /Ura⁻ /Lys⁻ /Ade⁺ synthetic dropout medium plates and expression of β-galactosidase activity.

### Example 2: In vitro cleavage of positive clone products by recombinant caspase-3

To construct expression plasmids for positive clones obtained from the two-hybrid screening, *Eco*RI*-Xho*I fragments of positive clones were inserted into the *Eco*RI*-Xho*I cloning sites of pYES2/NT-A plasmid (Invitrogen), in which the sequences were under control of the T7 promoter. Biotinylated lysine-labeled proteins were prepared from expression plasmids using a TNT T7 Quick Coupled Transcription/Translation System (Promega, Mannheim, Germany), according to the manufacturer's instructions. Five µl of biotinylated lysine-labeled protein were incubated for 1 h at 37 °C with 15 ng/µl recombinant active caspase-3 (BD PharMingen, Heidelberg, Germany) and optionally with 25 µM tetrapeptide caspase-3 inhibitor DEVD-fmk, in a Tris-CI reaction buffer, pH 7.5 (6 mM Tris-CI, pH 7.5, 1.2 mM CaCl₂, 5 mM DTT, 1.5 mM MgCl₂ and 1 mM KCI). The reaction was stopped by addition of SDS-PAGE sample buffer, and cleaved products were size fractionated by SDS-15% PAGE and blotted to a nitrocellulose membrane. Colorimetric detection of biotinylated products was performed on blots with Transcend Colorimetric Translation Detection System (Promega).

### Example 3: Antibodies and Western Blot Analysis

Rabbit ventricle protein extracts were prepared by homogenization in Tris-CI reaction buffer, pH 7.5. To examine the cleavage by caspase-3, 150 µg protein from control heart extracts were incubated for 1 hr at 37 °C with different amounts of recombinant human caspase-3, in presence or absence of the caspase-3 inhibitor DEVD-fmk (25 µM). After size-fractionation by SDS-PAGE, proteins were electrophoretically transfered to a nitrocellulose membrane and blots were incubated for 1 h at room temperature with mouse monoclonal antibodies against vMLC1 (0.2 µg/ml, clone 2c8, BiosPacific, Emeryville, California; 1:10 dilution, clone F109.16A12, Biocytex, Marseille, France), vMLC2 (1:10 dilution, clone F109.3E1, Biocytex), cardiac α/β myosin heavy chain (1:10 dilution, clone F26.4F4, Biocytex), α-sarcomeric actin (1:2,500 dilution, clone 5C5, Sigma, München, Germany) or cardiac Troponin T (0.4 µg/ml, clone 9B1, BiosPacific). Bound antibodies were detected with horseradish peroxidase-conjugated antibody against mouse IgG (1:10,000 dilution, Sigma) and visualized by chemiluminescence (ECL detection kit, Amersham Pharmacia, Freiburg, Germany).

### Example 4: Immunoprecipitation of vMLC1

Left ventricle lysates from control and 15 days paced failing male New Zealand white rabbits were prepared by homogenization in denaturing lysis buffer (50 mM Tris-Cl, pH 7.4, 5 mM EDTA, 1% SDS, 10 mM DTT, 1 mM PMSF, 2 µg/ml leupeptin and 15 U/ml DNase I), heated at 95 °C for 5 min, diluted 1:10 with nondenaturing lysis buffer (50 mM Tris-CI, pH 7.4, 300 mM NaCl, 5 mM EDTA, 1% Triton X-100, 10 mM iodoacetamide, 1 mM PMSF, 2 µg/ml leupeptin and 0.02% sodium azide) and centrifuged at 15,000 x g for 10 min at 4 °C. After dilution to 3.5 mg protein/ml, supernatants were precleared with excess of protein G-Sepharose beads (Sigma) and incubated for 2 h at 4 °C with protein G-Sepharose beads (30 µl beads/ml lysate), preconjugated with 100 µg anti-vMLC1 monoclonal antibody (clone 2c8, BiosPacific). Beads containing the immunocomplex were extensively washed with ice-cold nondenaturing lysis buffer, boiled in SDS sample buffer and subjected to SDS-15% PAGE and immunoblotting for vMLC1, as described above.

### Example 5: Model of Heart Failure

Medtronic pacemakers were implanted into New Zealand White rabbits (weight 3.6±0.3 Kg; from Harlan, Munich, Germany). Two days afterwards, rapid pacing was initiated at 320 beats/min. Under this protocol, a tachycardia-induced heart failure (HF) develops reproducibly over one week. Pacing was then continued at 360 beats/min, which predictably led to a further deterioriation of heart failure. The average contractility in failing hearts was 2200±320 mmHg/sec (vs. 4000±390 mmHg/sec in healthy controls; p<0.05), and LVEDP increased from 3.6±0.4 mmHg to 13.5±1.2 (p<0.05).

### Example 6: Preparation and culture of adult rabbit ventricular cardiomyocytes

Single myocytes were isolated from the left ventricle of control and 15 days paced failing rabbits, and cultured in M199 culture medium (supplemented with MEM vitamins, MEM non-essential aminoacids, 25 mM Hepes, 10 µg/ml insulin, 100 IU/ml penicillin, 100 µg/ml streptomycin and 100 µg/ml gentamicin) on laminin-precoated glass slides (5 µg/cm²; density of 10⁵ cells/cm²) in a humidified atmosphere (5% CO₂) at 37 °C (Laugwitz et al., 1999). Two hours after plating, cells were subjected to detection of activated caspase-3.

### Example 7: Activated Caspase-3 detection and fluorescence staining

Activated caspase-3 was detected in living cells by using CaspaTag Caspase-3 Activity Kit (Intergen, Oxford, United Kingdom), according to the manufacturer's instructions. Freshly isolated ventricular cardiomyocytes were incubated at 37°C (5% CO₂) with FAM-DEVD-fmk, a carboxyfluorescein labeled fluoromethyl ketone tetrapeptide inhibitor of caspase-3, which is cell permeable and irreversibly binds to activated caspase-3. After 1 h incubation, cells were washed, fixed in 4% paraformaldehyde, permeabilized in 100% methanol (at -20 °C) and subjected to Hoechst 33258 staining and either to immunofluorescence staining for vMLC1/vMLC2, or to phalloidin staining.
vMLCs were detected by labeling with specific mouse monoclonal antibodies anti-vMLC1 (4 µg/ml, clone 2c8, BiosPacific) and anti-vMLC2 (1:2 dilution, clone F109.3E1, Biocytex), followed by incubation with Texas Red goat anti-mouse-IgG conjugate (10 µg/ml, Molecular Probes, Leiden, The Netherlands).
Polymerized actin fibers were visualized by Texas Red-phalloidin (3 units/ml, Molecular Probes), according to the manufacturer's instructions.

### Example 8: Cell shortening experiments

Fractional shortening was measured in rabbit adult cardiomyocytes isolated from left ventricle of control and 15 days paced failing myocardium, after detection of activated caspase-3. Experiments were performed in a temperature-controlled cuvette (37 °C), at constant medium flow (1.8 mM Ca²⁺-Tyrode's solution) and constant electrical field, using an electro-optical monitoring system (Scientific Instruments, Heidelberg, Germany), as described (Laugwitz et al., 1999).

### Example 9: Myocardial Contractility Measurement by Echocardiography and Intraventricular Tip Catheterization

Left ventricular contractility was examined before the initiation of rapid pacing and at the end of the protocol (two weeks after the start of pacing). The rabbits were anesthetized as described before; ECG was monitored continuously.

### Example 10: Fluorescence Assay Development

The rate of caspase-3 enzyme activity could be measured by enzymatic cleavage and release of AMC from the Ac-DEVD-AMC caspase substrate (Biosyntan, Berlin, Germany). This parameter was measured as emission at 460 nm upon excitation at 380 nm using U.V. spectrofluorometry.

Ac-DFVE-AMC was synthesized by Biosyntan, Berlin, Germany, with a purity of 93.5%. The peptide lyophilised as trifluoracetic acid salt was reconstituted in DMSO to 100mM. 5 µg of purified, active recombinant human caspase-3 (CPP32) from BD Biosciences Pharmingen, Heidelberg, Germany, were diluted in 100µl 50 mM Tris, pH 8.0, with 100 mM NaCI, 50 mM imidazole.

The reaction buffer contained 20 mM HEPES, 100 mM NaCI, 10 mM DTT, 1 mM EDTA, 0.1 % (w / v) CHAPS, 10% sucrose, pH 7.2 and the indicated concentrations of Ac-DFVE-AMC. Caspase-3 was added to reaction mixture at a final concentration of 3 nM.

After preincubation for 10 min at 37°C, the released fluorogenic AMC was monitored every second minute for 20 min in a spectrofluorometer at an excitation wavelength of 380 nm and an emission wavelength of 460 nm.

Initial velocities and substrate concentrations were fit by non linear regression to the Michaelis-Menten equation. Lineweaver-Burk plots were calculated.

### References

Cesselli, D., I. Jakoniuk, L. Barlucchi, A.P. Beltrami, T.H. Hintze, B. Nadal-Ginard, J. Kajistura, A. Leri, and P. Anversa. 2001. Oxidative stress-mediated cardiac cell death is a major determinant of ventricular dysfunction and failure in dog dilated cardiomyopathy. *Cir. Res.* 89:279-286.

Chien, K.R. 2000. Genomic circuits and the integrative biology of cardiac diseases. *Nature* 407:227-232.

Cohen, G.M. 1997. Caspases: the executioners of apoptosis. *Biochem. J.* 326:1-16.

Haunstetter, A., and S. Izumo. 1998. Apoptosis: basic mechanisms and implications for cardiovascular disease. *Circ. Res.* 82:1111-1129.

Hengartner, M.O. 2000. The biochemestry of apoptosis. *Nature* 407:770-776.

Kamada, S., H. Kusano, H. Fujita, M. Ohtsu, R.C. Koya, N. Kuzumaki, and Y. Tsujimoto. 1998. A cloning method for caspase substrate that uses the yeast two-hybrid system: cloning of the antiapoptotic gene gelsolin. *Proc. Natl. Acad. Sci. USA.* 95:8532-8537.

Laugwitz, K.L., A. Moretti, H.J. Weig, A. Gillitzer, K. Pinkernell, T. Ott, I. Pragst, C. Städele, M. Seyfarth, A. Schömig, and M. Ungerer. 2001. Blocking caspase-activated apoptosis improves contractility in failing myocardium. *Hum. Gene Ther.* 12:2051-2063.

Laugwitz, K.L., M. Ungerer, T. Schöneberg, H.J. Weig, K. Kronsbein, A. Moretti, K. Hoffmann, M. Seyfarth, G. Schultz, and A. Schömig. 1999. Adenoviral gene transfer of the human V2 vasopressin receptor improves contractile force of rat cardiomyocytes. *Circulation* 99:925-933.

Lowey, S., G.S. Waller, and K.M.Trybus. 1993. Function of skeletal muscle myosin heavy and light chain isoforms by an in vitro motility assay. *J. Biol. Chem.* 268:20414-20418.

Mallat, Z., A. Tedgui, F. Fontaliran, R. Frank, M. Durigon, and G. Fontaine. 1996. Evidence of apoptosis in arrhythmogenic right ventricular dysplasia. *N. Engl. J. Med.* 335:1190-1196.

Milligan, R.A., M. Whittaker, and D. Safer. 1990. Molecular structure of F-actin and location of surface binding sites. *Nature.* 348:217-221.

Morano I, K. Hädicke, H. Haase, M. Böhm, E. Erdmann, and M.C. Schaub. 1997. Changes in essential myosin light chain isoform expression provide a molecular basis for isometric force regulation in the failing human heart. *J. Mol. Cell Cardiol.* 29:1177-1187.

Morano, I. 1999. Tuning the human heart molecular motors by myosin light chains. *J. Mol. Med.* 77:544-555.

Mittl PR, Di Marco S, Krebs JF, Bai X, Karanewsky DS, Priestle JP, Tomaselli KJ, Grutter MG. Structure of recombinant human CPP32 in complex with the tetrapeptide acetyl-Asp-Val-Ala-Asp fluoromethyl ketone. J Biol Chem. 1997 Mar 7;272(10):6539-47.

Narula, J., P. Pandey, E. Arbustini, N. Haider, N. Narula, F.D. Kolodgie, B. Dal Bello, M.J. Semigran, A. Bielsa-Masdeu, G.W. Dec, S. Israels, M. Ballester, R. Virmani, S. Saxena, and S. Kharbanda. 1999. Apoptosis in heart failure: Release of cytochrome c from mitochondria and activation of caspase-3 in human cardiomyopathy. *Proc. Natl. Acad. Sci. USA*. 96:8144-8149.

Nicholson, D.W., A. Ali, and N.A. Thornberry. 1995. Identification and inhibition of the ICE/CED-3 protease necessary for mammalian apoptosis. *Nature* 376:37-43.

Olivetti, G., R. Abbi, F. Quaini, J. Kajstura, W. Cheng, J.A. Nitahara, E. Quaini, C. De Loretto, C.A. Beltrami, S. Krajewski, T.C. Reed, and P. Anversa. 1997. Apoptosis in the failing human heart *N. Engl. J. Med.* 336:1131-1141.

Potter, K., H. Jiang, S. Hassanzadeh, S.R. Master, A. Chang, M.C. Dalakas, I. Rayment, J.R. Sellers, L. Fananapazir, and N.D. Epstein. 1996. Mutations in either the essential or regulatory light chains of myosin are associated with a rare myopathy in human heart and skeletal muscle. *Nat. Gen.* 13:63-69.

Price, K.M., W.A. Littler, and P. Cummins. 1980. Human atrial and ventricular myosin light chains subunits in the adult and during development. *Biochem. J.* 191:571-580.

Rayment, I., W.R. Rypniewski, K. Schmidt-Bäase, R. Smith, D.R. Tomchick, M.M. Benning, D.A. Winkelmann, G. Wesenberg, and H.M. Holden. 1993. Three-dimensional structure of myosin subfragment-1: a molecular motor. *Science.* 261:50-58.

Schaub, M.C., and H.O. Hirzel. 1987. Atrial and ventricular isomyosin composition in patients with different forms of cardiac hypertrophy. *Basic Res. Cardiol*. 82(Suppl.2):357-367.

Sütsch, G., U.T. Brunner, C. von Schulthess, H.O. Hirzel, O.M. Hess, M. Turina, H.P. Krayenbuehl, and M.C. Schaub. 1992. Hemodynamic performance and myosin light chain-1 expression in the hypertrophied left ventricle in aortic valve disease before and after valve replacement. *Cir. Res.* 70:1035-1043.

Sweeney, H.L., 1995. Function of the N terminus of the myosin essential light chain of vertebrate striated muscle. *Biophys. J.* 68(Suppl.4):112S-119S.

Talanian RV, Quinlan C, Trautz S, Hackett MC, Mankovich JA, Banach D, Ghayur T, Brady KD, Wong WW. Substrate specificities of caspase family proteases. J Biol Chem. 1997 Apr 11;272(15):9677-82.

Trayer, I.P., H.R. Trayer, and B.A. Levine. 1987. Evidence that the N-terminal region of A1-light chain of myosin interacts directly with the C-terminal region of actin. A proton magnetic resonance study. *Eur. J. Biochem.* 164:259-266.

VanBuren, P., G.S. Waller, D.E. Harris, K.M. Trybus, D.M. Warshaw, and S. Lowey. 1994. The essential light chain is required for full force production by skeletal muscle myosin. *Proc. Natl. Acad. Sci. USA.* 91:12403-12407.

Vemuri, R., E.B. Lankford, K. Potter, S. Hassanzadaeh, K. Takeda, Z.X. Yu, V.J. Ferrans, and N.D. Epstein, 1999. The stretch-activation responce may be critical to the proper funtioning of the mammalian heart. *Proc. Natl. Acad. Sci. USA.* 96:1048-1053.

Wei Y, Fox T, Chambers SP, Sintchak J, Coll JT, Golec JM, Swenson L, Wilson KP, Charifson PS. The structures of caspases-1, -3, -7 and -8 reveal the basis for substrate and inhibitor selectivity. Chem Biol. 2000 Jun;7(6):423-32.

Yin, X., S. Gu, and J.X. Jiang. 2001. The development-associated cleavage of lens connexin 45.6 by caspase-3-like protease is regulated by casein kinase II-mediated phosphorylation. *J. Biol. Chem.* 276:34567-34572.

## Claims

1. Use of a peptide containing an essential ventricular myosin light chain type 1 (vMLC1) amino acid sequence, which is functional as cleavage site for caspase-3, in the screening for a compound for the treatment of chronic or acute cardiovascular disease.

2. Use according to claim 1, wherein the amino acid sequence is DFVE.

3. Use according to claim 1 or 2, wherein the peptide is vMLC1.

4. Use according to any one of the preceding claims wherein the screening is directed to a compound which selectively inhibits the caspase-3-mediated cleavage of vMLC1 under predetermined conditions while essentially not inhibiting the caspase-3-mediated cleavage of a protein containing a functional caspase-3 DEVD cleavage site under the same conditions.

5. Use according to claim 4, wherein the selectivity is based on the structure of the compound.

6. Use according to claim 4, wherein the selectivity of the compound is based on the concentration of the compound.

7. A screening method for inhibitors of the caspase-3-mediated cleavage of vMLC1, which comprises:
(a) contacting a test compound and a sample containing
(i) a peptide containing a vMLC1 amino acid sequence which is functional as cleavage site for caspase-3, and
(ii) caspase-3,
under predetermined conditions allowing cleavage of the peptide at the cleavage site in the absence of the test compound, followed by
(b) determining the presence or absence of an inhibition of the protein cleavage activity at the cleavage site as compared to the absence of the test compound, and
(c) identifying a compound as an inhibitor which provides for the presence of inhibition of the caspase-3-mediated cleavage of the protein in step (b).

8. A screening method for selective inhibitors of the caspase-3-mediated cleavage of vMLC1 over the caspase-3-mediated cleavage of a peptide containing a functional caspase-3 DEVD cleavage site, which comprises:
(a) contacting a predetermined amount of an inhibitor identified or identifiable by the screening method of claim 7 and a sample containing
(i) a peptide containing a functional caspase-3 DEVD cleavage site,
(ii) caspase-3, and optionally
(iii) a peptide containing a functional caspase-3 vMLC1 cleavage site,
under predetermined conditions allowing cleavage of a peptide containing a functional caspase-3 vMLC1 cleavage site in the absence of the test compound, followed by
(b) determining the presence or absence of a change of the protein cleavage activity at the cleavage site of the peptide containing a functional caspase-3 DEVD cleavage site as compared to the absence of the test compound, and
(c) identifying a compound as a selective inhibitor which provides at the predetermined concentration for an essential absence of a change of the protein cleavage activity at the cleavage site of the peptide containing a functional caspase-3 DEVD cleavage site.

9. The method of claim 7, wherein the screening method for selective inhibitors of the caspase-3-mediated cleavage of vMLC1 over the caspase-3-mediated cleavage of a peptide containing a functional caspase-3 DEVD cleavage site of claim 8 is simultaneously carried out.

10. The method of any one of claims 7 to 9, wherein the peptide containing a vMLC1 amino acid sequence which is functional as cleavage site for caspase-3 is vMLC1.

11. The method of any one of claims 7 to 10, wherein the peptide contains the sequence DFVE as amino acid sequence of essential ventricular myosin light chain which is functional as cleavage site for caspase-3.

12. A cell assay for screening for inhibitors of the caspase-3-mediated cleavage of vMLC1, which comprises
(a) providing a culture of isolated cardiomyocytes,
(b) introducing activated caspase-3 into cardiomyocytes of step (a),
(c) determining the presence or absence of a reduction of the extent of caspase-3-mediated cleavage of vMLC1 and/or an improvement of cell contractility under predetermined conditions in the presence of a test compound as compared to the absence of the test compound,
(d) identifying a compound as an inhibitor which provides for the presence of inhibition of the caspase-3-mediated cleavage of vMLC1 and/or for an improved cell contractility in step (c).

13. A cell assay for screening for selective inhibitors of the caspase-3-mediated cleavage of vMLC1 over the caspase-3-mediated cleavage of a peptide containing a functional caspase-3 DEVD cleavage site, which comprises
(a) providing a culture of isolated cardiomyocytes,
(b) introducing activated caspase-3 into cardiomyocytes of step (a),
(c) determining the presence or absence of a change of the extent of protein cleavage at the cleavage site of the peptide containing a functional caspase-3 DEVD cleavage site in the presence of a predetermined amount of an inhibitor identified or identifiable by the assay of claim 12 as compared to the absence of the inhibitor, and
(c) identifying a compound as a selective inhibitor which provides in the predetermined amount for an essential absence of a change of the protein cleavage at the cleavage site of the peptide containing a functional caspase-3 DEVD cleavage site.

14. The assay of claims 12, wherein the assay for screening for selective inhibitors of the caspase-3-mediated cleavage of vMLC1 over the caspase-3-mediated cleavage of a peptide containing a functional caspase-3 DEVD cleavage site of claim 13 is simultaneously carried out.

15. An *in vivo* assay for screening for inhibitors of the caspase-3-mediated cleavage of vMLC1, which comprises
(a) providing an animal model, preferably for heart failure,
(b) administering a test compound to the animal model of step (a),
(c) determining the presence or absence of a reduction of the extent of caspase-3-mediated cleavage of vMLC1 and/or an improvement of heart failure under predetermined conditions in the presence of the test compound as compared to the absence of the test compound,
(d) identifying a compound as an inhibitor which provides for the presence of inhibition of the caspase-3-mediated cleavage of vMLC1 and/or for an improvement of heart failure in step (c).

16. An *in vivo* assay for screening for selective inhibitors of the caspase-3-mediated cleavage of vMLC1 over the caspase-3-mediated cleavage of a peptide containing a functional caspase-3 DEVD cleavage site, which comprises
(a) providing an animal model, preferably for heart failure,
(b) administering a test compound to the animal model of step (a),
(c) determining the presence or absence of a change of the extent of protein cleavage at the cleavage site of the peptide containing a functional caspase-3 DEVD cleavage site in the presence of a predetermined amount of an inhibitor identified or identifiable by the assay of one of claims 7 to 15 as compared to the absence of the inhibitor, and
(d) identifying a compound as a selective inhibitor which provides in the predetermined amount for an essential absence of a change of the protein cleavage activity at the cleavage site of the peptide containing a functional caspase-3 DEVD cleavage site.

17. The assay of claims 15, wherein the assay for screening for selective inhibitors of the caspase-3-mediated cleavage of vMLC1 over the caspase-3-mediated cleavage of a peptide containing a functional caspase-3 DEVD cleavage site of claim 16 is simultaneously carried out.

18. The assay of any one of claims 15 to 17, wherein the determination in step (c) is performed based on a measurement of contractility of cardiomyocytes and/or Western blotting.

19. The assay of claim 12 or 15, wherein the reduction in the extent of caspase-3-mediated cleavage of vMLC1 is determined by detection of a specific cleavage product of caspase-3-mediated cleavage of vMLC1, notably by Western blotting.

20. Kit-of-parts for identifying inhibitors of the caspase-3-mediated cleavage of vMLC1 according to claim 7, comprising the following components:
(i) a first component comprising a peptide containing an essential ventricular myosin light chain amino acid sequence, which is functional as cleavage site for caspase-3, and
(ii) a second component comprising caspase-3.

21. Kit-of-parts for identifying selective inhibitors of the caspase-3-mediated cleavage of vMLC1 over the caspase-3-mediated cleavage of a peptide containing a functional caspase-3 DEVD cleavage site according to claim 8, comprising the following components:
(i) a first component comprising a peptide containing a functional caspase-3 DEVD cleavage site,
(ii) a second component containing caspase-3, and optionally
(iii) a third component comprising a peptide containing a functional caspase-3 vMLC1 cleavage site.

22. Inhibitor of caspase-3-mediated cleavage of essential ventricular myosin light chain obtained or obtainable by the method of any one of claims 1 to 19.

23. The inhibitor according to claim 22, which is a selective inhibitor of the caspase-3-mediated cleavage of vMLC1 over the caspase-3-mediated cleavage of a peptide containing a functional caspase-3 DEVD cleavage site.

24. The inhibitor according to claim 22, which is a peptide containing the sequence DFVE, or a derivative thereof.

25. Use of the inhibitor according to any one of claims 22 to 24 for the preparation of a medicament for the treatment of chronic cardiovascular disease.

26. Medicine containing as an active agent a compound which is **characterized by** inhibiting caspase-3-mediated cleavage of vMLC1.

27. Peptide containing the sequence DFVE as amino acid sequence of essential myosin light chain which is functional as cleavage site for caspase-3, with the exception of native essential myosin light chain.
